# EUROPEAN PATENT SPECIFICATION

(11) **EP 1 767 242 B1**
(45) Date of publication and mention of the grant of the patent: **20.07.2011**
(21) Application number: 05749236.5
(22) Date of filing: 12.05.2005
(51) Int. Cl.: A61M 39/22, A61F 2/48, A61M 25/10

(54) **VALVE FOR AUTOMATICALLY REGULATING VESICAL EMPTYING IN CATHETERISED PATIENTS**
VENTIL FÜR DIE AUTOMATISCHE REGULIERUNG DER VESIKALEN ENTLEERUNG IN KATHETERISIERTEN PATIENTEN
SOUPAPE DESTINEE A REGULER AUTOMATIQUEMENT LE VIDAGE VESICAL CHEZ DES PATIENTS CATHETERISES

(30) Priority: 09.06.2004 ES 200401401
(43) Date of publication of application: 28.03.2007
(73) Proprietor: Conejero Sugranes, Joan, 08021 Barcelona (ES); Gamissans Bou, Marius, 17500 Ripoll (Gerona) (ES); Graus Albarracin, Jorge, 08025 Barcelona (ES)
(72) Inventor: Conejero Sugranes, Joan, 08021 Barcelona (ES); Gamissans Bou, Marius, 17500 Ripoll (Gerona) (ES); Graus Albarracin, Jorge, 08025 Barcelona (ES)
(74) Representative: Pons Ariño, Angel
(86) International application number: PCT/ES2005/000259
(87) International publication number: WO 2005/123174

(56) References cited:
- WO-A1-01/10358
- WO-A1-03/030986
- ES-A1- 8 400 668
- ES-A1- 8 505 255
- ES-A6- 2 025 946
- ES-B1- 2 081 233
- ES-T1- 2 107 991
- ES-T3- 2 148 687
- ES-T3- 2 166 836
- ES-T3- 2 193 044
- ES-Y- 291 529
- GB-A- 1 154 623
- US-A- 3 570 484
- US-A- 5 727 594
- US-A- 5 967 490
- US-B1- 6 267 564

## Description

### OBJECT OF THE INVENTION

The present invention refers to a valve to automatically regulate vesicular emptying in catheterised patients.

### BACKGROUND OF THE INVENTION

The permanent vesical catheter is currently used in different therapies, for example, in patients with vesical emptying problems, either neurologically caused or not and in men or women.

The use of the catheter may be open, so that the draining or outlet of urine is constant; or with a stopper or element to interrupt the flow, each of them having their disadvantages.

As disadvantages in open use, it is necessary to mention the following:
- The urinary bladder loses its capacity to distend on always remaining empty.
- The infection itself that any permanent catheter involves produces structural changes in the vesical wall, the elastic fibres in the detrusor being replaced by collagen fibres, causing fibrosis; this fibrosis may cause stenosis of the urethral meatuses with ureterohydronephrosis and renal compromise with variable degrees of renal insufficiency.
- The permanent catheter itself may cause vesical spasms with urine outlet around the probe and the patient getting wet in spite of having their permanent catheter.

As disadvantages of the use of the probe with a flow-interrupting element, the following must be mentioned:
- It may not be opened when asleep, so the disadvantages are the same as in the previous modality.
- The opening of the catheter has to be carried out regularly and at specific times, when the person is not there most of the time or the moment to do it is not convenient.
- The opening of the catheter may lead to urinary infections through contamination on its handling.
- The opening of the stopper implies wetting the hands with urine if one is not sufficiently skilful.
- In tetraplegic patients, a third person has to carry out the above-mentioned opening.
- With the flow interrupted, high pressure caused in the bladder may produce deterioration in the upper urinary section and urine leaks around the catheter.
- The emptying rate must match the diuresis.

In order to resolve these inconveniences, the applicants are request and transfer holders of Spanish patent n °. 9200577, regarding a valve for intermittent vesical emptying.

This patent protects a valve insertable in the urinary circuit by means of connectors, that is to say, in the catheter, inside which more or less complex means are fitted, with buoys and inner deposits to open and allow urine circulation when the pressure in the bladder exceeds values of 30 g/cm2 or their equivalent of 22 mmHg/cm2, closing in a timed way by means of internally structured droppers.

This valve, though operative, has certain disadvantages due to its own configuration:
- The internal urine flow is very slow, making the opening of the valve difficult to carry out.
- It has a greater volume and weight.
- Functioning only works in vertical or almost upright positions, due to the existence of the internal buoy.
- The timing of opening does not guarantee the complete emptying of the bladder.
- Larger structural complexity, which implies less reliability and higher cost.
- Existence of gaskets at the elastic joints, implying the same disadvantage as in the previous case.

US patent US5727594, upon which the preamble of claim 1 is based, describes a unidirectional flow valve for medical purposes, which has a low actuation pressure and allows a continuous flow at low pressures. The valve disclosed in US5727594 comprises a housing including a fluid flow passage extending therethrough provided with a first housing half and a second housing half. The valve also comprises an elastomeric valve member acting as a flow stop member and defining a passage between said valve member and the housing.

The valve disclosed in US5727594 allows continuous flow for low pressures, in particular for pressures which are substantially lower than the pressure which is needed in the bladder to begin urinating, i.e. approximately 30 gr/cm² for humans. Therefore it is not able to reproduce the act of urinating.

### DESCRIPTION OF THE INVENTION

The invented valve has a simple, economic embodiment that solves all the aforementioned inconveniences.

In accordance with the invention, the valve comprises a tubular valve body whose ends stretch out forming two opposite connectors to be inserted into the urinary circuit, between the bladder and collection bag.

The above-mentioned body has a widening which forms an inner housing where a plunger runs, shutting the inlet by means of a return element, preferably a spring, suitably set to produce the force needed to maintain the valve closed while the urine pressure P1 multiplied by the surface of the obturating element this pressure is exerted on, corresponding to section s1 of the inlet connector, does not exceed setting value F of the return element means.

That is:
While P1 x s1 <F closed valve.
When P1 x s1> = F the valve opens.

When the second condition occurs, the plunger moves towards the outlet connector, its stroke being limited by stops that prevent this connector closing.

The urine then flows through a space provided between the plunger and its housing. A necessary, main condition in the invention is that the flow section in this space is narrower than the flow sections in both connectors to provoke strangulation on the urine passing through when the valve is open that causes a residual pressure upon the whole head of the plunger which keeps the valve open. By suitably sizing the dimensions and/or connector sections, plunger and housing, as well as the characteristics of the return element, the valve will remain open until the complete emptying of the bladder, since, though the urine flow pressure drops during urination, the surface on which the residual pressure is exerted on (the whole head of the plunger) is considerably higher than the surface it is exerted on when the valve is closed.

Analytically explaining the previous argument, if there is a residual pressure P2 during the opening of the valve that is then exerted on the whole surface of the obturating element s2:
While P2 x s2 > F valve open.
When P2 x s2 <= F the valve closes.

Ideally surface s2 will be from 7 to 15 times larger than s1.

Immediately after the closing of the valve, the cycle begins again. The value of P1 is then almost 0 (bladder empty) and it begins to raise, the valve being closed until this value reaches approximately the value of 30 mm. water column value, which is the value that is medically considered to be the maximum to which the bladder may be subjected. The return element means are set so that when P1 exceeds this value, the valve opens again.

In this way, a complete emptying of the bladder is obtained, since it is not timed but through residual pressure, in such a way that a different catheter dimensioning will cause emptying time to vary, but it will always be complete.

As described above, with the use of the valve according to the present invention it is possible to reproduce the act of urinating, because the valve has different opening and closing pressures, due to the presence of the passage defined between the plunger and the housing, whose section is narrower than the sections in both connector, thus allowing the complete emptying of the bladder.

### BRIEF DESCRIPTION OF THE DRAWINGS

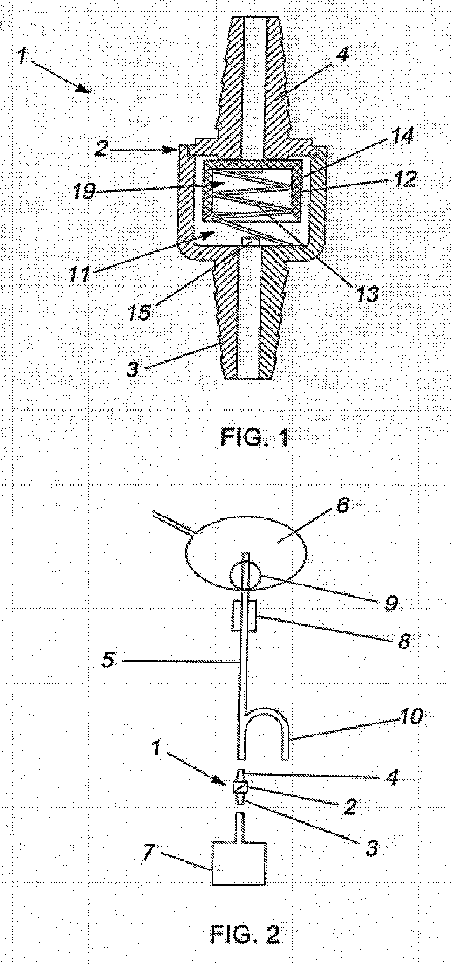

Figure 1 shows a view of the insertion of the invented valve into the urinary circuit.
Figure 2 shows a diametrical section of the invented valve.
Figure 3 shows a view of figure 2 where the valve appears with both its operating conditions, open and closed.
Figure 4 shows a detail of the adjustment between both parts forming the body of the invented valve.

### DESCRIPTION OF A PRACTICAL EMBODIMENT OF THE INVENTION

The invented valve 1 comprises a tubular body 2 with two end connectors 3,4 that are opposite and axially aligned, to be inserted into any part of the catheter or urinary circuit 5 between the bladder 6 and external collection bag 7, inserting the catheter through the urethra 8, being retained by the ball 9 that inflates through the valve 10.

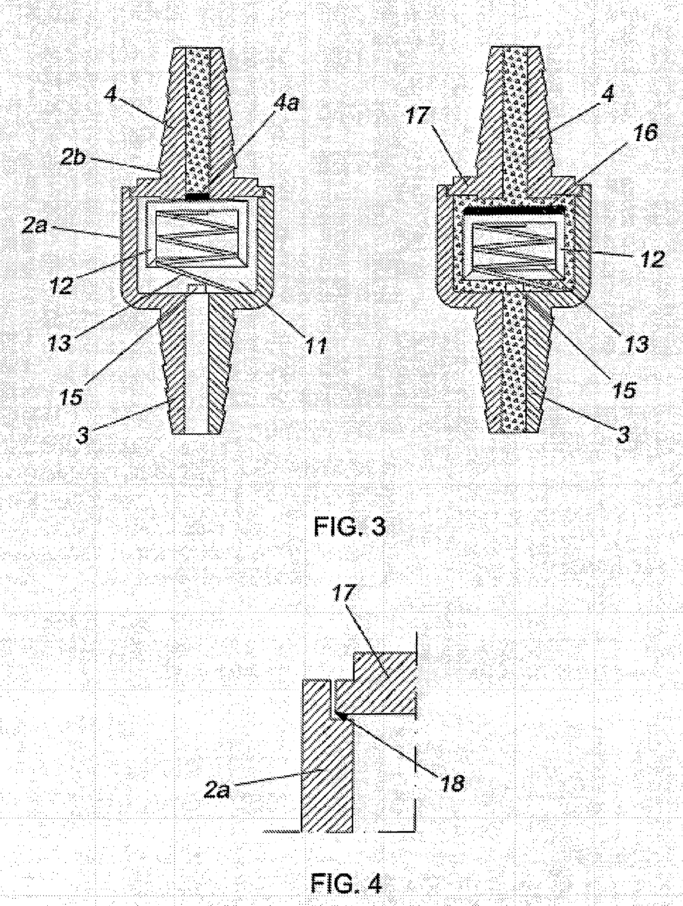

The body 2 has a central widening which forms an inner housing 11 for a plunger 12 that closes the inlet connector 4, thanks to the action of a return spring 13. The urine pressure, as seen in figure 3, when the valve is closed, is only exerted on section 4a of the plunger head in the lengthening of the inner section of the inlet connector 4. To trigger the opening of the valve, this pressure has to reach 30 g/cm2, the medically advisable value.

The plunger 12 has a smaller section than that of the housing 11, so that the urine may flow through the space 14 existing between both on the piston moving and opening the inlet connector. Likewise, stops 15 are fitted inside the housing 11 that limit the stroke of the plunger to maintain a separation from the outlet connector 4 and to allow urine to flow through it.

The space 14 has a smaller section than the inlet and outlet connectors' inner section, producing strangulation and deceleration in flow, leading to a residual pressure being exerted on the whole head of the plunger 16 and able to keep the valve open until the complete emptying of the bladder, though urine pressure drops below initial opening values.

In this non-restrictive example of the invention, the body 2 comprises two parts, 2a + 2b, assembled by fitting them together. One of them, 2a, has a cylindrical, tubular bell shape, fitted with the outlet connector 3, housing 11 and the stops 15, whereas the other, 2b, includes the inlet connector 4 and a head 17 that fits into the mouth of the housing 11, its bottom resting on a perimetral recess 18 made on its inside.

As for the plunger, it is cylindrical and has a lower box-shaped recess 19 to house the upper top part of the spring 13, stabilizing its fixture.

## Claims

1. Valve (1) to automatically regulate vesical emptying in catheterised patients; of the type comprising a tubular body (2) with two end connectors (3, 4) to be inserted into the urinary circuit (5), with a widening that contains flow-cutting elements (12, 13), these flow-cutting elements (12, 13) comprising a plunger (12) with return element (13) that blocks the inlet connector (4) inside, and runs inside a housing (11) fitted inside the widening of the tubular body (2), the head of the plunger (12) having a larger surface than the inner section of the inlet connector (4), and plunger stroke stops (15) being fitted in order to provide a lower space that enables urine flow to enter the outlet connector (3) on the plunger (12); wherein between the plunger (12) and the housing (11) inside which it moves, is a urine passage space (14), **characterised by** said passage space (14) being narrower than the inlet (4) and outlet connector (3) sections, in order to produce urine flow deceleration in this area during valve-opening, causing a residual pressure to be exerted upon the whole head of the plunger (12) keeping the valve (1) open until the force exerted by this pressure drops below the value of that exerted in the opposite direction by the return element (13), thus causing the valve (1) to close.

## Patentansprüche

1. Ventil (1) zur automatischen Regulierung der Blasenentleerung bei katheterisierten Patienten; von der Art, die ein röhrenförmiges Gehäuse (2) mit zwei Endkonnektoren (3, 4) zur Einführung in den Harnkreislauf (5) umfasst, mit einer Verbreiterung, die den Durchfluss unterbrechende Elemente (12, 13) enthält, wobei diese den Durchfluss unterbrechenden Elemente (12, 13) einen Kolben (12) mit einem Rückstellelement (13) umfassen, das den Einlasskonnektor (4) innen sperrt und innerhalb eines Gehäuses (11) verläuft, das in die Verbreiterung des röhrenförmigen Gehäuses (2) eingepasst ist, wobei der Kopf des Kolbens (12) eine größere Oberfläche als den Innenquerschnitt des Einlasskonnektors (4) besitzt und Kolbenhubanschläge (15) montiert sind, um einen niedrigeren Raum bereitzustellen, welcher es dem Harnfluss ermöglicht, in den Auslasskonnektor (3) auf dem Kolben (12) zu gelangen; wobei zwischen dem Kolben (12) und dem Gehäuse (11), in dem sich dieser bewegt, ein Harndurchlassraum (14) vorgesehen ist, **dadurch gekennzeichnet, dass** der besagte Durchlassraum (14) enger als die Querschnitte des Einlasskonnektors (4) und Auslasskonnektors (3) ist, um in diesem Bereich eine Verlangsamung des Harnflusses während der Öffnung des Ventils zu erzeugen, wodurch ein Restdruck auf den gesamten Kopf des Kolbens (12) aufgebracht wird, welcher das Ventil (1) offen hält, bis die von diesem Druck ausgeübte Kraft unter den Wert der von dem Rückstellelement (13) in die entgegengesetzte Richtung ausgeübten Kraft fällt, wodurch das Ventil (1) dazu gebracht wird, sich zu schließen.

## Revendications

1. Clapet (1) pour réguler automatiquement la vidange vésicale chez les patients cathétérisés ; du type comprenant un corps tubulaire (2) avec deux raccords d'extrémité (3, 4) devant être insérés dans le circuit urinaire (5), avec un élargissement qui contient des éléments de coupure de flux (12, 13), ces éléments de coupure de flux (12, 13) comprenant un piston (12) avec un élément de retour (13) qui bloque le raccord d'entrée (4) à l'intérieur, et passe à l'intérieur d'un logement (11) placé à l'intérieur de l'élargissement du corps tubulaire (2), la tête du piston (12) ayant une surface plus grande que la section interne du raccord d'entrée (4), et des arrêts de course de piston (15) étant disposés de façon à créer un espace inférieur qui permet au flux d'urine d'entrer dans le raccord de sortie (3) sur le piston (12) ; où entre le piston (12) et le logement (11) à l'intérieur duquel il bouge, se trouve un espace de passage pour l'urine (14), **caractérisé par** ledit espace de passage (14) qui est plus étroit que les sections des raccords d'entrée (4) et de sortie (3), de façon à produire la décélération du flux d'urine dans cette zone durant l'ouverture du clapet, ce qui a pour conséquence d'exercer une pression résiduelle sur toute la tête du piston (12) gardant le clapet (1) ouvert jusqu'à ce que la force exercée par cette pression tombe en dessous de la valeur de celle exercée dans la direction opposée par l'élément de retour (13), provoquant ainsi la fermeture du clapet (1).
